# EUROPÄISCHE PATENTANMELDUNG

(11) **EP 0 624 402 A2**
(43) Veröffentlichungstag der Anmeldung: **17.11.1994**
(21) Anmeldenummer: 94104057.8
(22) Anmeldetag: 16.03.1994
(51) Int. Cl.: B05C 11/10, B05C 5/02, A61L 15/00, A61L 15/32

(54) **Vorrichtung zum gleichmässigen Auftragen einer Suspension auf einen Kollagenträger**

(30) Priorität: 31.03.1993 AT 647/93
(71) Anmelder: Nycomed Arzneimittel GmbH, D-80939 München (DE)
(72) Erfinder: Hagedorn, Olaf, D-48231 Warendorf (DE); Schiele, Ulrich, Dr., D-80805 München (DE)

(57) **Zusammenfassung**

Vorrichtung zum gleichmäßigen Auftragen einer Suspension auf einen Kollagenträger zur Herstellung eines Materials zum Abdichten und Heilen von Wunden, die aus einem Behälter, in welchen die Suspension eingebracht wird, aufgebaut ist, dessen Boden aus einem Grundrahmen (1) und einer Bodenlochplatte (2) besteht, wobei direkt über der Bodenlochplatte (2) eine bewegliche Lochplatte (3), angebracht ist.

## Beschreibung

Die Erfindung betrifft eine Vorrichtung zum gleichmäßigen Auftragen einer Suspension auf einen Kollagenträger zur Herstellung eines Materials zum Abdichten und Heilen von Wunden.

Aus EP 0 059 265 ist ein Material zum Abdichten und Heilen von Wunden bekannt, das aus einem Kollagenträger besteht, der mit einer Fibrinkomponente, einer Thrombinkomponente und Zusätzen, wie zum Beispiel Calciumionen, Proteaseinhibitoren oder Heparinantagonisten, beschichtet ist.

Zur Herstellung dieses Materials werden die einzelnen Komponenten bzw. Zusätze in einem organischen Lösungsmittel, wie etwa Ethanol, suspendiert und anschließend auf den Kollagenträger, etwa durch Besprühen, aufgebracht.

Dabei treten jedoch mehrere Probleme auf, da die aufzutragende Suspension schwer handzuhaben ist. So verstopfen die für solche Zwecke verwendeten Flüssigkeitsdüsen sofort. Flüssigkeits-Luft-Düsen erlauben größere Durchmesser, aber auch hier ermöglichen nur Düsen mit den größten angebotenen Durchmessern hinreichend störungsfreies Arbeiten. Diese Düsen haben jedoch einen entscheidenden Nachteil. Durch die unscharfe Abgrenzung des Austrittstrahls wird keine gleichmäßige Suspensionsschicht, sondern ein trapezförmiges Beschichtungsprofil auf dem Kollagenträger erzeugt. Dies führt zu erheblichen Randverlusten an Kollagenträger bzw. wertvoller Suspension.

Aus EP-A 472 050 ist eine Vorrichtung zum Auftragen eines Flüssigkeitsfilms auf eine Warenbahn nach dem Ausgießprinzip bekannt, die über einzelne sich unmittelbar nebeneinander erstreckende Flüssigkeitsverteilräume eine zwangsweise Verteilung der Flüssigkeit von einer Zuflußöffnung bis zu einer Vielzahl von Ausflußöffnungen bewirkt. Die Verteilung der Flüssigkeit erfolgt in der Form eines Stammbaumes, nämlich schrittweise von einer Öffnung auf zwei, vier, acht, sechzehn usw. Ausflußöffnungen. Diese Vorrichtung ist für das gleichmäßige Verteilen eines Suspension, die eine Fibrinogenkomponente und eine Thrombinkompente enthält, nicht geeignet, da durch die mehrmalige Teilung der Flüssigkeitsströme ein Verkleben und Verstopfen der Flüssigkeitsräume durch die Suspension erfolgt und stärker auftritt als bei der Verwendung von Düsen.

Aufgabe der vorliegenden Erfindung war es deshalb die oben genannten Nachteile der bisher bekannten Methoden zu verhindern.
Diese Aufgabe konnte nun mit der erfindungsgemäßen Vorrichtung gelöst werden.
Gegenstand der Erfindung ist daher eine Vorrichtung zum gleichmäßigen Auftragen einer Suspension auf einen Kollagenträger zur Herstellung eines Materials zum Abdichten und Heilen von Wunden, die dadurch gekennzeichnet ist, daß die Vorrichtung aus einem Behälter, in welchen die Suspension eingebracht wird, aufgebaut ist, dessen Boden aus einem Grundrahmen (1) und einer Bodenlochplatte (2) besteht, wobei direkt über der Bodenlochplatte (2) eine bewegliche Lochplatte (3), angebracht ist.

Der Behälter besitzt eine von einem Grundrahmen (1) umgebene rechtwinkelige Lochplatte (Bodenlochplatte (2)) auf dem sich seitliche Begrenzungswände befinden. Unmittelbar über der Bodenlochplatte (2) liegt eine zweite Lochplatte auf, die innerhalb des Behälters hin- und herbewegt werden kann: bewegliche Lochplatte (3).

Die in den Behälter einzubringende Suspension besteht aus einer Fibrinkomponente, einer Thrombinkomponente, sowie aus Aprotinin als Proteaseinhibitor und anderen Zusätzen, wie beispielsweise Calciumionen oder Heparinantagonisten, in einem Alkohol, wie Ethanol, n-oder i-Propanol, oder n- oder i-Butanol. Sie wird zur Herstellung eines Materials zum Abdichten und Heilen von Wunden und somit für medizinische Zwecke verwendet. Aus diesem Grund muß die Vorrichtung, insbesonders die Lochplatten, aus einem abriebfesten Material, das mit der Suspension nicht in Reaktion treten kann, gefertigt sein. Geeignete Materialien sind beispielsweise Edelstahl oder Titan. Die seitlichen Begrenzungswände können auch aus Glas bzw. Plexiglas aufgebaut sein, wodurch die Suspension im Behälter gut beobachtet werden kann.

Beide Lochplatten weisen jeweils eine oder mehrere Lochreihen auf, wobei die Durchflußlöcher in den Lochreihen jeweils im gleichen Abstand angeordnet sind. Vorzugsweise befinden sich mehrere Lochreihen auf den Lochplatten. Der Durchmesser der Durchflußlöcher soll groß genug gewählt werden, um ein Verstopfen der Durchflußlöcher mit der Suspension zu verhindern.
Das Verhältnis des Durchmessers der Durchflußlöcher zu dem Durchmesser der größten Partikel der Suspension beträgt etwa 5 : 1 bis 50 : 1, bevorzugt 7,5 : 1 bis 40 : 1 und besonders bevorzugt 10 : 1 bis 30 : 1.
Die größten in der Suspension vorhandenen Partikel besitzen einen Durchmesser von etwa 0,1 mm bis 0,2 mm.
In einer bevorzugten Ausführungsform der erfindungsgemäßen Vorrichtung beträgt der Durchmesser der Durchflußlöcher etwa 2 bis 3 mm.

Die Mittelpunkte der Durchflußlöcher liegen bevorzugt in einem Abstand zwischen 2 bis 8 mm, besonders bevorzugt in einem Abstand von 3 - 4,5 mm quer zur Front der Beschichtung.
Je nach Zahl der Lochreihen kann der Abstand zwischen zwei benachbarten Löchern in einer Lochreihe jedoch bis zu 16 mm und mehr betragen.

Zum Auftragen der Suspension auf den Kollagenträger wird die vorher homogenisierte Suspension mit einer konstanten Pumpgeschwindigkeit in den Behälter gepumpt, wobei anfangs die bewegliche Lochplatte so auf der Bodenlochplatte liegt, daß die Durchflußlöcher geschlossen sind. Die Abdichtung soll dabei möglichst effizient sein und kann gegebenenfalls durch Auflagegewichte auf der beweglichen Lochplatte unterstützt werden.
Wenn die Suspension im Behälter das der vorgegebenen Pumpgeschwindigkeit entsprechende, stationäre Niveau erreicht hat, wird die Beschichtungsapparatur in Gang gesetzt. Dadurch wird die bewegliche Lochplatte über der stationären Lochplatte hin und her bewegt. In einer Position - bevorzugt in der Mitte zwischen beiden Umkehrpunkten, an denen die Richtungsänderung der beweglichen Lochplatte stattfindet - kommen beide Lochplatten zur Deckung und Suspension kann auf den Kollagenträger tropfen, der auf einem Förderband unter der Beschichtungsapparatur hindurchgeführt wird.
Das Niveau der Suspension in der Beschichtungsapparatur bleibt dabei konstant solange neue Suspension zugepumpt wird.

Durch verschieden weite Auslenkung der beweglichen Lochplatte kann das Verhältnis von geöffnetem zu geschlossenem Zeitintervall in weiten Grenzen eingestellt werden. Es ist dadurch möglich, Lochdurchmesser von einer Größe zu wählen, bei welcher keine Komplikationen mehr auftreten und gleichzeitig die Auftropfgeschwindigkeit zu begrenzen.

Unter Berücksichtigung von Lochreihenanordnung und Geschwindigkeit des Förderbandes läßt sich ein Verteilungsmuster der Tropfen erzielen, in dem die Tropfen die Ecken gleichseitiger Dreiecke bilden, das einer dichtest gepackten Kugelschicht entspricht.

Die Bewegung der Lochplatte erfolgt bevorzugt im rechten Winkel zur Transportrichtung des Förderbandes.
Durch die ständige Hin- und Herbewegung der beweglichen Lochplatte wird die Suspension gleichzeitig homogen gehalten, sodaß eine gleichmäßige Verteilung der Komponenten auf dem Kollagenträger erzielt wird. Gegebenenfalls kann die Vermischung durch zusätzliche Anordnungen auf der beweglichen Lochplatte oder mittels eines Rührers unterstützt werden.

Eine weitere Ausführungsform besteht darin, daß anstatt der Platten, zwei ineinander rotierende Rohre bzw. ein Rohr und ein Halbrohr verwendet werden. Das äußere Rohr bzw. Halbrohr ist bevorzugt starr angeordnet und nach unten mit Austrittsöffnungen versehen. In dieses Rohr ist ein inneres Rohr eingepaßt, welches mit Lochreihen versehen ist, die mit den Ausflußöffnungen des äußeren Rohres zur Deckung kommen können. Durch periodisches Hin-und Herdrehen des inneren Rohres bzw Halbrohres werden die Ausflußöffnungen periodisch geöffnet und geschlossen, womit der angestrebte Effekt erreicht wird.

Mit Hilfe der erfindungsgemäßen Vorrichtung ist es möglich, die Suspension gleichmäßig in einer genau definierten Breite auf den Kollagenträger aufzutragen, ohne daß Randverluste an Suspension bzw. an Kollagenträger auftreten.
Das nach dem Verdampfen des Suspensionsmedium erhaltene Beschichtungsprofil ist nicht, wie bisher mit bekannten Besprühungstechniken erzielt, trapezförmig, sondern rechteckig.

Ein Vergleichsversuch, in welchem der Randverlust, der mit der bisher angewandten Besprühungstechnik mittels einer Flüssigkeits-Luft-Düse erhalten wird, dem Verlust, der mit der erfindungsgemäßen Vorrichtung erhalten wird, gegenübergestellt wurde, zeigt, daß mit der Flüssigkeits-Luft-Düse mehr als 5 mal so viel an Suspensionsflüssigkeit verloren geht, als mit der erfindungsgemäßen Vorrichtung.
Dabei handelt es sich um einen relativ kleinen Produktionsansatz. Das Verhältnis wird umso größer, je größer die Ansätze sind.
Der Verlust an Suspension bei der Auftragung mit der erfindungsgemäßen Vorrichtung ist dabei nur durch das nach Beendigung des Zupumpens verbleibende Restvolumen an Suspension im Behälter bedingt.

### Beispiel 1:

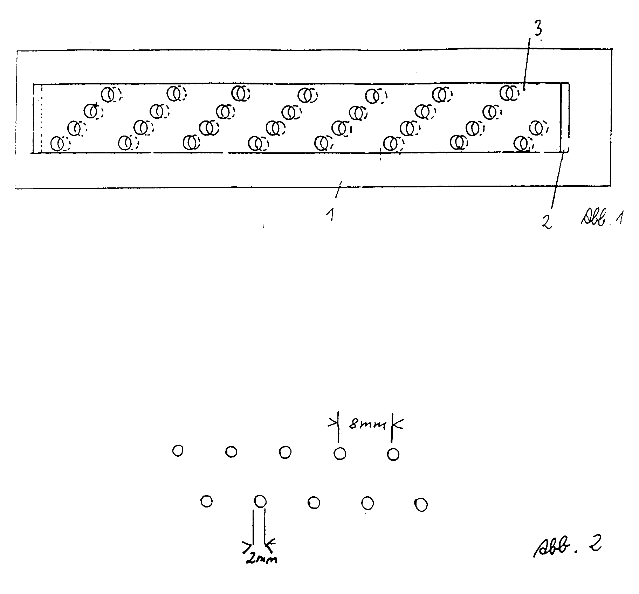

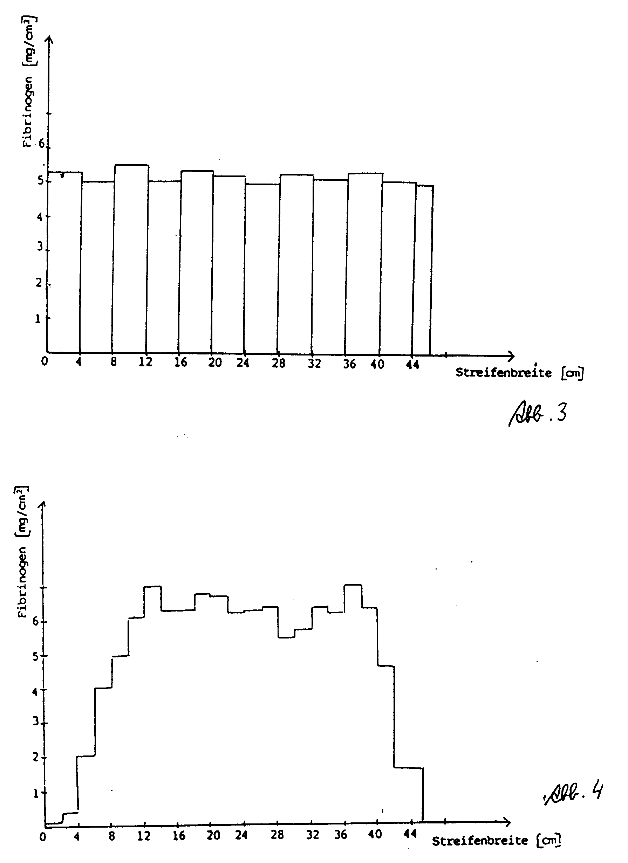

In einen Behälter mit einer Bodenlochplatte und einer beweglichen Lochplatte, die folgende Abmessungen aufwiesen:
Breite:450 mm
Tiefe: 12 mm
Lochreihenanzahl: 2
Durchflußlochdurchmesser: 2 mm
Abstand zwischen den Mittelpunkten zweier Durchflußlöcher in einer Reihe: 8 mm
Abstand zwischen den Lochreihen: 6,9 mm
(Die Durchflußlochanordnung ist in Abbildung 2 dargestellt.)
wurde bei geschlossenen Lochplatten eine Suspension aus 55 mg/ml Fibrinogen 20 IU/ml Thrombin und 0,71 Ph. Eur. U./ml Aprotinin in Ethanol mit einer Geschwindigkeit von 450 ml/min gepumpt, bis die stationäre Flüssigkeitshöhe von 50 mm erreicht wurde. In diesem Moment wurde die bewegliche Lochplatte mit 400 Perioden/min in Bewegung gesetzt, wobei die Auslenkung 6 mm in beide Richtungen betrug.
Die Suspension tropfte dabei in 450 mm Breite auf einen Kollagenschwamm von 5 mm Höhe, der mittels eines Förderbandes mit einer Geschwindigkeit von 1 m/min im rechten Winkel zur Bewegung der beweglichen Lochplatte unter dem Behälter hindurch transportiert wurde. Nach Verdampfen der Suspensionsflüssigkeit war der Kollagenträger mit etwa 5,5 mg/cm² Fibrinogen 2 IU/cm² Thrombin und 0,071 Ph.Eur. U./cm² Aprotinin beschichtet.
Der Randverlust lag unter 1 %.

Das Beschichtungsprofil quer zur Transportrichtung des Förderbandes ist in Abbildung 3 dargestellt.

### Vergleichsbeispiel:

Es wurden wiederum eine Suspension gleicher Zusammensetzung in 450 mm Breite auf einen Kollagenträger aufgebracht der mit einem Förderband transportiert wurde. Abb. 4 zeigt das beste Ergebnis zahlreicher Versuche mit verschiedenen Flüssigkeit-Luft-Düsen.
In diesem Beispiel wurde eine Düsenkombination von Spraying Systems Inc. mit Umlenkfläche verwendet. Aus zahlreichen Mustern desselben Types wurde das beste Exemplar ausgewählt.

Das Beschichtungsprofil quer zur Transpsortrichtung des Förderbandes ist in Abbildung 4 dargestellt, und zeigt die Verteilung von Fibrinogen quer zur Transportrichtung des Förderbandes. Der Verlust durch die an beiden Seiten abfallende Menge beträgt unter diesen Bedingungen immer noch ca. 20 %.

## Patentansprüche

1. Vorrichtung zum gleichmäßigen Auftragen einer Suspension auf einen Kollagenträger zur Herstellung eines Materials zum Abdichten und Heilen von Wunden, dadurch gekennzeichnet, daß die Vorrichtung aus einem Behälter, in welchen die Suspension eingebracht wird, aufgebaut ist, dessen Boden aus einem Grundrahmen (1) und einer Bodenlochplatte (2) besteht, wobei direkt über der Bodenlochplatte (2) eine bewegliche Lochplatte (3), angebracht ist.

2. Vorrichtung nach Anspruch 1, dadurch gekennzeichnet, daß die Bodenlochplatte (2) und die bewegliche Lochplatte (3) jeweils mindestens eine Reihe äquidistant angeordneter Durchflußlöcher besitzen.

3. Vorrichtung nach Anspruch 1, dadurch gekennzeichnet, daß die Bodenlochplatte (2) und die bewegliche Lochplatte (3) mehr als eine Durchflußlochreihe aufweisen, wobei die Mittelpunkte der Löcher quer zur Laufrichtung des Förderbandes in einem Abstand zwischen 2 und 8 mm bevorzugt 3 bis 4,5 mm versetzt sein sollen.

4. Vorrichtung nach Anspruch 1, dadurch gekennzeichnet, daß das Verhältnis der Durchmesser der Durchflußlöcher zu den Durchmessern der größten Partikel der Suspension mindestens 5 : 1 bis maximal 50 : 1, bevorzugt 7,5 : 1 bis 40 : 1 und besonders bevorzugt 10 : 1 bis 30 : 1 beträgt.

5. Vorrichtung nach Anspruch 1, dadurch gekennzeichnet, daß der Fluß der Suspension durch die Einstellung der Auslenkung der beweglichen Lochplatte (3) regulierbar ist.

6. Vorrichtung nach Anspruch 1, dadurch gekennzeichnet, daß der Behälter in Form eines Rohres oder Halbrohres ausgebildet ist, das nach unten mit Austrittsöffnungen versehen ist, in das ein bewegliches perforiertes hin-und herbewegbares Rohr bzw. Halbrohr eingepaßt ist.
